# EUROPEAN PATENT SPECIFICATION

(11) **EP 3 903 684 B1**
(45) Date of publication and mention of the grant of the patent: **24.04.2024**
(21) Application number: 19901416.8
(22) Date of filing: 12.11.2019
(51) Int. Cl.: A61B 7/04, H04R 1/46, H04R 17/02, A61B 5/33, A61B 5/28

(54) **STETHOSCOPE AND ELECTRONIC STETHOSCOPE DEVICE**
STETHOSKOP UND ELEKTRONISCHE STETHOSKOPVORRICHTUNG
STÉTHOSCOPE ET DISPOSITIF DE DISPOSITIF ÉLECTRONIQUE

(30) Priority: 26.12.2018 JP 2018243492
(43) Date of publication of application: 03.11.2021
(73) Proprietor: FUJIFILM Corporation, Tokyo 106-8620 (JP)
(72) Inventor: FUJII Takamichi, Ashigarakami-gun, Kanagawa 258-8577 (JP); SASAKI Tsutomu, Ashigarakami-gun, Kanagawa 258-8577 (JP)
(74) Representative: Dewhurst, Joseph Michael
(86) International application number: PCT/JP2019/044435
(87) International publication number: WO 2020/137212

(56) References cited:
- WO-A1-2018/020887
- WO-A1-2021/020203
- WO-A2-2015/170772
- JP-A- H10 309 272
- JP-A- 2010 534 098
- JP-A- 2014 090 916
- JP-A- 2017 153 640
- US-A1- 2013 310 709
- US-A1- 2013 310 709

## Description

### BACKGROUND OF THE INVENTION

### 1. Field of the Invention

The present invention relates to a stethoscope and an electronic auscultation apparatus capable of measuring a body sound with good reproducibility.

### 2. Description of the Related Art

In general, a stethoscope has been used as an instrument for observing a living body from the outside. The stethoscope can amplify sounds such as heart sounds or blood flow sounds generated inside the living body to directly listen to the sounds. In recent years, an electronic auscultation system has been proposed in which sounds from the stethoscope are connected with a computer through a digital conversion device or the like and are recorded by using the computer.

The electronic auscultation system has been expected to be used in a remote diagnosis. In the remote diagnosis using a videophone or the like, in a case of making a diagnosis using a stethoscope, a method has been used in which a patient himself or herself brings an auscultation portion into contact with his or her own body, and the body sounds are detected by a sound sensor, are converted into electrical signals, and are transmitted to a doctor who is at a remote location through communication means such as the Internet. JP2017-170112A proposes a configuration in which the auscultation portion is provided with a sensor that detects contact with a human body in order to make it possible to recognize from a remote location that the auscultation portion is in proper contact with the body surface.

Further, from the viewpoint that the detected signals by a pulse wave sensor that detects pulse waves vary depending on a pressing force between the pulse wave sensor and the human body in an electronic watch type pulse wave processing device, WO94/015525A discloses a configuration in which a pressing force detection unit that confirms whether or not to be in a contact state with an appropriate pressing force is provided.

In general, the stethoscope has a portion that is put to the skin (auscultation portion), and the auscultation portion is provided with a diaphragm for collecting sounds. Meanwhile, the stethoscope in the electronic auscultation system is known to have a configuration in which a diaphragm and a sound (vibration) sensor with an air layer therebetween are provided in the auscultation portion (for example, JP2016-179177A). On the other hand, JP2012-090909A proposes a stethoscope comprising a sound sensor that includes a piezoelectric element acquiring body sounds directly from the skin without the air layer. According to the stethoscope of JP2012-090909A, since the piezoelectric element is directly put to the skin, the detectable frequencies can be greatly expanded. Specifically, the sound sensor of JP2012-090909A includes a metal plate of which one surface comes into contact with the skin of the human body, and a piezoelectric ceramic formed on the other surface of the metal plate.

US 2013/0310709 discloses a stethoscope according to the preamble of independent claim 1.

### SUMMARY OF THE INVENTION

In the piezoelectric element of JP2012-090909A, the portion that comes into contact with the skin has a flat surface, and the detection efficiency cannot be said to be sufficient. It is desirable that the stethoscope can efficiently detect very low body sounds.

The present invention has been made in view of the above circumstances, and an object thereof is to provide a stethoscope and an electronic auscultation apparatus capable of measuring a body sound with a high signal to noise (SN) ratio.

Specific means for solving the above problems include the following aspects.
<1> The present invention, which is defined in claim 1, is a stethoscope comprising: a support base; and a detection unit that is supported by the support base and detects a sound generated from an object to be measured, wherein the detection unit has a piezoelectric film that is disposed to face the support base in at least a portion for detecting the sound generated from the object to be measured and that is convexly curved to a side opposite to the support base, the piezoelectric film includes a piezoelectric layer having two main surfaces facing each other, a first electrode provided on a main surface on a support base side of the two main surfaces, and a second electrode provided on a main surface on a side opposite to the support base, the second electrode is provided only in a central part of a convexly curved portion of the piezoelectric film, and a strain generated in the piezoelectric film due to the sound generated from the object to be measured is detected as a vibration signal. Preferred embodiments are detailed below:
<2> The stethoscope according to <1>, further comprising a sound insulation member that blocks an external sound, which is provided around the portion for detecting the sound generated from the object to be measured.
<3> The stethoscope according to <2>, in which the sound insulation member is formed of an elastic member having a portion protruding further outward than an apex of a convexly curved portion of the piezoelectric film.
<4> The stethoscope according to any one of <1> to <3>, further comprising a cushioning material between the support base and the piezoelectric film.
<5> The stethoscope according to any one of <1> to <3>, further comprising a pressurized gas in a space between the support base and the piezoelectric film.
<6> The stethoscope according to any one of <1> to <5>, in which the piezoelectric layer consists of a polymer composite piezoelectric body obtained by dispersing piezoelectric particles in a matrix consisting of a polymer material.
<7> The stethoscope according to any one of <1> to <6>, further comprising an electrocardiography electrode.
<8> The stethoscope according to <7>, in which the electrocardiography electrode is provided on a surface side of the piezoelectric film provided with the second electrode.
<9> The stethoscope according to <8>, in which the electrocardiography electrode and the second electrode each are formed of a patterned electrode layer.
<10> The stethoscope according to any one of <1> to <9>, further comprising a pressure sensor that detects a contact pressure of the piezoelectric film to the object to be measured, between the support base and the piezoelectric film.
<11> The stethoscope according to any one of <1> to <10>, further comprising a processing unit that performs at least one of first processing of obtaining a contact pressure to the object to be measured having a maximum amplitude of the vibration signal detected with the piezoelectric film or second processing of determining whether or not a contact pressure to the object to be measured is adequate.
<12> The stethoscope according to <11>, in which the processing unit performs processing of causing a notification unit to make notification of at least one of the pressure obtained in the first processing or a determination result determined in the second processing.
<13> An electronic auscultation apparatus comprising: the stethoscope according to any one of <1> to <12>; and a processing device that receives a vibration signal and data regarding a contact pressure detected by the stethoscope, in which the processing device performs at least one of first processing of obtaining a contact pressure to the object to be measured having a maximum amplitude of the vibration signal detected with the piezoelectric film or second processing of determining whether or not a contact pressure to the object to be measured is adequate, from the vibration signal and the data regarding the contact pressure.

With the stethoscope and the electronic auscultation apparatus according to the present disclosure, the body sound can be measured with a high SN ratio.

### BRIEF DESCRIPTION OF THE DRAWINGS

Fig. 1 is a schematic view showing a configuration of a stethoscope according to a first embodiment.
Fig. 2 is a diagram for explaining a method of calculating a radius of curvature of a piezoelectric film.
Fig. 3A is an enlarged view showing a cross-section of the piezoelectric film.
Fig. 3B is an enlarged view showing a cross-section of another piezoelectric film in a design modification example.
Fig. 4 is a schematic view showing a configuration of a stethoscope according to a second embodiment.
Fig. 5 is a schematic view showing a state in which the stethoscope according to the second embodiment is in contact with a skin surface of a living body.
Fig. 6 is a schematic view showing a configuration of a stethoscope according to a third embodiment.
Fig. 7 is a block diagram showing the configuration of the stethoscope according to the third embodiment.
Fig. 8 is a schematic view showing a configuration of a stethoscope according to a fourth embodiment.
Fig. 9 is a block diagram showing the configuration of the stethoscope according to the fourth embodiment.
Fig. 10 is a schematic view showing a configuration of a stethoscope according to a fifth embodiment.
Fig. 11 is a schematic view showing a configuration of a stethoscope according to a sixth embodiment.
Fig. 12 is a graph each showing that an SN ratio of a signal varies depending on the magnitude of a contact pressure.

### DESCRIPTION OF THE PREFERRED EMBODIMENTS

Hereinafter, embodiments of a stethoscope according to the present disclosure will be described with reference to the drawings.

### "Stethoscope of First Embodiment"

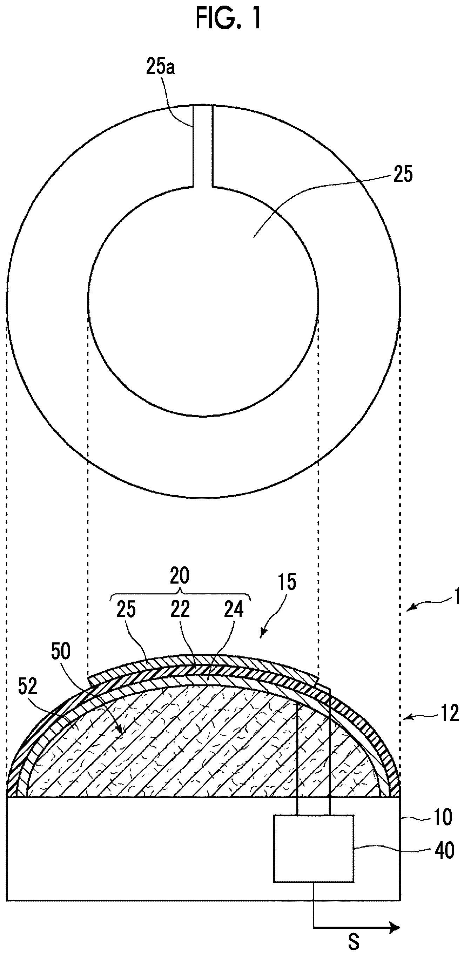

Fig. 1 is a schematic plan view showing a stethoscope 1 according to a first embodiment of the present invention and a schematic side view showing a part thereof as a cross-section. For easy visibility in the drawing, the film thickness of each layer and ratio thereof are appropriately modified and drawn, and do not necessarily reflect the actual film thickness and ratio (the same applies to the following drawings).

The stethoscope 1 of the present embodiment comprises a support base 10 and a detection unit 12 supported by the support base 10. The detection unit 12 detects a sound generated from an object to be measured. The detection unit 12 has a piezoelectric film 20 disposed to face the support base 10 and convexly curved to a side opposite to the support base 10. The piezoelectric film 20 comprises a piezoelectric layer 22 having two main surfaces facing each other, a first electrode 24 provided on a main surface on a support base 10 side of the two main surfaces, and a second electrode 25 provided on a main surface on a side opposite to the support base 10. The piezoelectric film 20 may be provided in at least a portion for detecting the sound generated from the object to be measured (hereinafter, also referred to as a sensor region 15).

For example, the stethoscope 1 has the same size as that of an auscultation portion (chest piece) of the conventional stethoscope.

With the above configuration, the stethoscope 1 detects a strain generated in the piezoelectric film 20 due to the sound generated from the object to be measured, as a vibration signal. Specifically, in a case of making the piezoelectric film 20 of the sensor region 15 come into contact with the object to be measured, the piezoelectric film 20 vibrates with the vibration generated on the surface of the object to be measured due to the sound generated from the object to be measured, and the vibration is detected as the voltage generated between the first electrode 24 and the second electrode 25. In the present specification, the voltage detected in this way is referred to as a vibration signal. The stethoscope 1 comprises a detection circuit 40, as a detector that is connected to the first electrode 24 and the second electrode 25 of the piezoelectric film 20 and that detects the voltage between both the electrodes 24 and 25. The detection circuit 40 may be provided inside the support base 10 or outside the support base 10. The detection circuit 40 is provided with an amplifier circuit that amplifies the vibration signal, and the vibration signal is amplified and output from the detection circuit 40. The amplified vibration signal S is converted into an acoustic signal (sound) by a conversion unit that converts the signal into the sound, and can be heard from the speaker. Alternatively, it can be heard from earpieces through simulated eustachian tubes, as in the conventional stethoscope structure. The support base 10 is a portion that a user grips when the stethoscope 1 is pressed against the object to be measured, and the shape and the material thereof are not limited. The piezoelectric film 20 is provided so as to face at least a part of the support base 10. The piezoelectric film 20 can be regarded as being convexly curved as long as the piezoelectric film 20 is supported in a state in which a constant tension is applied so as to be convex outward with a space between the part of the support base 10 and the piezoelectric film 20.

The piezoelectric film 20 may be directly connected to a part of the support base 10, or may be connected to the support base 10 through another member.

In the following, the object to be measured is a living body, and the sound generated from the object to be measured will be described as a sound generated from the living body (body sound). Examples of the sounds generated from the living body include heartbeat, respiratory sounds, blood vessel sounds, and intestinal sounds. Note that, the stethoscope according to the present disclosure can also be used to detect abnormal sounds of a machine, an abnormality in piping, and the like. That is, the object to be measured includes not only a living body but also a machine and piping, and the sound generated from the object to be measured includes sounds generated from the machine, the piping, and the like.

The body sounds can be detected with a high SN ratio by pressing the convexly curved piezoelectric film 20 of the stethoscope 1 against the skin surface of a living body, that is, the skin with an appropriate pressing pressure. A displacement (vibration) of the body surface caused by the body sounds can be directly detected with the piezoelectric film 20 pressed against the skin. Therefore, the noise component can be suppressed, as compared with the conventional stethoscope that has a built-in microphone and detects vibration through an air layer. In addition, detectable frequencies can be greatly expanded. Further, the detection efficiency of body sounds can be improved by pressing the curved piezoelectric film 20 against the skin, as compared with a case of pressing a non-curved flat piezoelectric film against the skin. A strain is generated in the piezoelectric film 20 due to the vibration of the body surface, the strain is converted into the voltage in the piezoelectric film 20, and the voltage is detected as the vibration signal. In a case where the piezoelectric film is provided in a curved shape, expansion and contraction in the in-plane direction generated in the piezoelectric layer when the strain occurs can be increased as compared with a case where the piezoelectric film is provided in a non-curved flat shape. Therefore, the amplitude of the obtained voltage becomes large. Accordingly, it is possible to collect sounds with a high SN ratio.

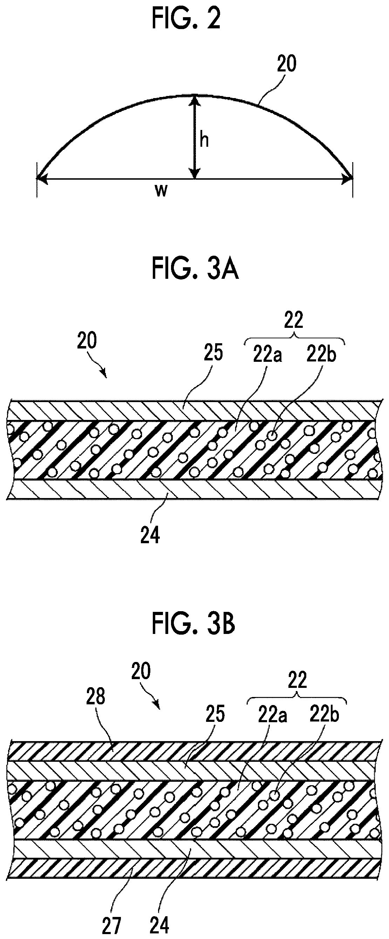

The degree of curvature of the piezoelectric film can be expressed by the radius of curvature thereof. As shown in Fig. 2, the piezoelectric film 20 is assumed to be supported by the support base in a state in which the piezoelectric film 20 forms an arc having a uniform radius of curvature, and the radius of curvature is obtained by the equation R = {(w/2)² + h²} / 2h. Here, the radius of curvature of the piezoelectric film 20 is denoted by R, a distance (camber) from a surface including connection ends with the support base to the apex of a convex portion is denoted by h, and in the surface including the connection ends with the support base, a longest length between the connection ends, which passes through the point intersecting h is denoted by w (see Fig. 2). w corresponds to, for example, the diameter of a disc in a case where a disc-shaped support base is provided.

For example, in a case of assuming a chest piece-shaped stethoscope having a diameter of about 3 to 10 cm, the radius of curvature of the piezoelectric film is preferably 1 m or less, more preferably 0.6 m or less, from the viewpoint of sufficiently obtaining the effect caused by making the piezoelectric film curved. On the other hand, the radius of curvature is preferably more than 0.03 m from the viewpoint of achieving high sensitivity while ensuring a sufficient contact area with the skin.

In the piezoelectric film 20, the vicinity of the apex of the convex portion may be a flat portion having a substantially flat shape, the outer edge connected to the flat portion may form an inclined portion starting from the support base 10, and the connected portion of the flat portion and the inclined portion may have a greatly curved shape. In a case of having such a shape, the radius of curvature R of the piezoelectric film 20 is also obtained from the distance h to the apex of the convex portion and the longest length w between the ends of the piezoelectric film in the same manner as described above.

The piezoelectric film 20 may be provided in at least a portion for detecting sound generated from an object to be measured, but may be provided in the entire surface facing the support base 10. In the stethoscope 1 of the present embodiment, a space 50 between the piezoelectric film 20 and the support base 10 is filled with a cushioning material 52. In the stethoscope 1, the cushioning material 52 makes the piezoelectric film 20 convexly curved and supported in a state in which a certain tension is applied.

The cushioning material 52 has an appropriate elasticity, and is provided so as to support the piezoelectric film 20 and to improve the efficiency of charge generation by applying a constant mechanical bias to any part of the piezoelectric film 20 and then converting the movement (vibration) in a direction perpendicular to the film surface of the piezoelectric film 20 into the expansion and contraction movement in the in-plane direction of the film.

Further, a stethoscope having an appropriate repulsive force can be achieved by changing the filling density of the cushioning material.

The cushioning material 52 is not limited, as long as the cushioning material 52 has an appropriate elasticity and is suitably deformed without hindering the strain from being generated in the piezoelectric film 20. Specifically, it is preferable to use fiber materials such as wool felt containing polyester fibers such as rayon and polyethylene terephthalate (PET) and glass wool, and foaming materials such as polyurethane.

Instead of filling the space 50 with the cushioning material 52, the space 50 may be filled with a pressurized gas such as the pressurized air.

### [Piezoelectric film]

As shown by enlarging a part of the piezoelectric film 20 in Fig. 3A, the piezoelectric film 20 has a piezoelectric layer 22, a first electrode 24 provided on one surface of the piezoelectric layer 22, and a second electrode 25 provided on the other surface. The piezoelectric film 20 has elasticity and flexibility to the extent that cracks do not occur when the piezoelectric film 20 is pressed against the object to be measured. As shown in Fig. 3B, the piezoelectric film 20 may further have a protective layer 27 provided on the surface of the first electrode 24 and a protective layer 28 provided on the surface of the second electrode 25. As the piezoelectric layer 22, a piezoelectric layer in which expansion and contraction occur in the in-plane direction, that is, the main surface thereof expands and contracts in a case of applying a voltage between the first electrode 24 and the second electrode 25 is used.

The material of the piezoelectric layer 22 may be polyvinylidene fluoride (PVDF), vinylidene-trifluoroethylene copolymer (P(VDF-TrFE)), an organic piezoelectric film such as polylactic acid, and a polymer composite piezoelectric body disclosed in JP2014-199888A. As shown in Figs. 3A and 3B, the polymer composite piezoelectric body is obtained by uniformly dispersing the piezoelectric particles 22b in a matrix 22a containing a polymer material. Further, the piezoelectric layer 22 needs to be subjected to poling (polarization).

The piezoelectric particles 22b in the piezoelectric layer 22 may be regularly or irregularly dispersed in the matrix 22a.

As the matrix 22a, for example, a polymer material having viscoelasticity at a normal temperature, such as cyanoethylated polyvinyl alcohol (cyanoethylated PVA), is preferable. Examples of the matrix 22a include polyvinyl acetate, polyvinylidene chloride-co-acrylonitrile, a polystyrene-vinyl polyisoprene block copolymer, poly vinyl methyl ketone, and poly butyl methacrylate, in addition to the cyanoethylated PVA.

The piezoelectric particles 22b are piezoelectric particles. The piezoelectric particle 22b is preferably a ceramic particle having a perovskite-type crystal structure. Examples of the ceramic particle include lead zirconate titanate, lead lanthanum zirconate titanate, barium titanate, and a solid solution of barium titanate and bismuth ferrite.

Further, as the material of the piezoelectric layer 22, a polymer electret material containing, as a main component, a polymer disclosed in JP2018-191394A, JP2014-233688A, and JP2017-012270A, for example, an organic material such as polyimide; polypropylene; Teflon (registered trademark) such as polytetrafluoroethylene (PTFE (tetra fluoride)), a tetrafluoroethylene/perfluoroalkyl vinyl ether copolymer (PFA), a tetrafluoroethylene/hexafluoropropylene copolymer (FEP (tetra/hexa fluoride)), and an amorphous fluoropolymer (AF); polyethylene; and cycloolefin polymers (COCs) can also be used.

The first electrode 24 and the second electrode 25 are electrodes for detecting the strain of the piezoelectric layer 22 as a voltage.

The materials for forming the first electrode 24 and the second electrode 25 are not particularly limited, and various conductors can be used. Specific examples of the materials include C, Pd, Fe, Sn, Al, Ni, Pt, Au, Ag, Cu, Cr, Mo, and the like, and an alloy thereof. Further, a transparent conductive film such as indium tin oxide (ITO), indium zinc oxide (IZO), tin oxide, and zinc oxide can be used. Furthermore, an organic conductor such as a conductive polymer can also be used. The method of forming the electrode is also not particularly limited, and various known methods such as a film formation by a vapor phase deposition method (vacuum film forming method) such as vacuum evaporation or sputtering, screen printing, and a method of attaching a foil made of the above material can be available.

Among them, in particular, a thin film of copper or aluminum, or a conductive polymer formed by vacuum evaporation is suitability used as the first electrode 24 and the second electrode 25 because the flexibility of the piezoelectric film 20, that is, the magnitude of the backward and forward movement can be ensured and a thin electrode layer which does not restrict the deformation of the piezoelectric layer can be formed.

The thickness of each of the first electrode 24 and the second electrode 25 is not particularly limited, but is preferably 1 µm or less and preferably as thin as possible. Further, the thicknesses of the first and second electrodes 24 and 25 are basically the same, but may be different from each other.

Further, the first electrode 24 and/or the second electrode 25 does not necessarily need to be formed so as to correspond to the entire surface of the piezoelectric layer 22. That is, at least one of the first electrode 24 or the second electrode 25 may be smaller than, for example, the piezoelectric layer 22. The second electrode 25 provided on the surface on the convex side is provided only in the central part of the convexly curved portion of the piezoelectric film 20.

The central part of the convexly curved portion of the piezoelectric film 20 includes the apex of the convexly curved portion of the surface on the convex side, and is preferably a region in which the distance from the apex is equal to or less than half the distance from the apex to the connection end with the support base.

The protective layers 27 and 28 are not particularly limited, and various sheet-like materials can be used. As an example, various resin films (plastic films) are suitably exemplified. Among them, for reasons such as excellent mechanical strength and heat resistance, polyethylene terephthalate (PET), polypropylene (PP), polystyrene (PS), polycarbonate (PC), polyphenylene sulfide (PPS), polymethylmethacrylate (PMMA), polyetherimide (PEI), polyimide (PI), polyethylene naphthalate (PN), triacetyl cellulose (TAC), polyethylene naphthalate (PEN), a cyclic olefin resin, a natural rubber, a fluorosilicone rubber, and a silicone rubber are suitably used.

The thicknesses of the protective layers 27 and 28 are also not particularly limited. The thicknesses of the protective layers 27 and 28 are basically the same, but may be different from each other.

Here, as with the electrodes 24 and 25 described above, high stiffness of the protective layers 27 and 28 may restrict expansion and contraction of the piezoelectric layer 22, and as a result, the amplitude of the backward and forward movement of the piezoelectric film may become small. Therefore, the thicknesses of the protective layers 27 and 28 may be appropriately set according to the performance, handleability, mechanical strength, and the like required for the piezoelectric film 20, that is, the sound sensor.

A part of the piezoelectric film 20 is provided with wiring for connecting the first electrode 24 and the second electrode 25 to the detection circuit 40. The schematic side view of Fig. 1 schematically shows the connection lines connecting the first electrode 24 and the second electrode 25 to the detection circuit 40. For example, as shown in the plan view of Fig. 1, the wiring 25a connected to the second electrode 25 is provided so as to extend to the end portion of the piezoelectric film 20. The wiring 25a and the second electrode 25 can be formed by patterning the electrode layer at the same time. In this case, the wiring 25a and the second electrode 25 are made of the same material.

With the stethoscope 1 of the present embodiment, it is possible to measure internal sounds of the body emitted from the living body, such as heartbeat, respiratory sounds, blood vessel sounds, and intestinal sounds, with high accuracy. For example, abnormal sounds in the heartbeat caused by aortic stenosis, pulmonary stenosis, mitral insufficiency, tricuspid insufficiency, and the like can be detected accurately. Further, for example, it is possible to acquire data corresponding to the blood pressure by measuring blood vessel sounds in a case of attaching the stethoscope 1 to the arm or the like, and sufficiently strongly pressing and slowly releasing the blood vessel.

The stethoscope 1 shown in Fig. 1 can be used as a chest piece portion in the conventional stethoscope. The stethoscope 1 is provided with a tube which has incorporating electric wires for transmitting signals from the detection circuit, eustachian tubes, and earpieces, and thereby can be used in the same manner as the conventional stethoscope. Further, the stethoscope 1 may be provided with a function for wireless communication such as Bluetooth (registered trademark) and infrared communication so as to be capable of communicating with a smartphone, a personal computer, or the like. The detected data can be transmitted to a display unit and a signal processing unit of a smartphone or a personal computer, and then waveform can be confirmed on a monitor or used for a diagnosis of changes over time by being recorded as data.

With the stethoscope 1, the sensor region has a convex shape. Therefore, it can be achieved that the piezoelectric film comes into good contact with the skin surface, and body sounds can be detected with high efficiency. On the other hand, since the sensor region has a convex shape, a gap between the portion around the convex of the detection unit 12 and the living body is generated and external sounds including environmental sounds, human voice, and the like easily enter from the gap when the sensor region is pressed against the skin. Therefore, it is preferable to provide means for eliminating the influence of noise caused by the external sounds.

### "Stethoscope of Second Embodiment"

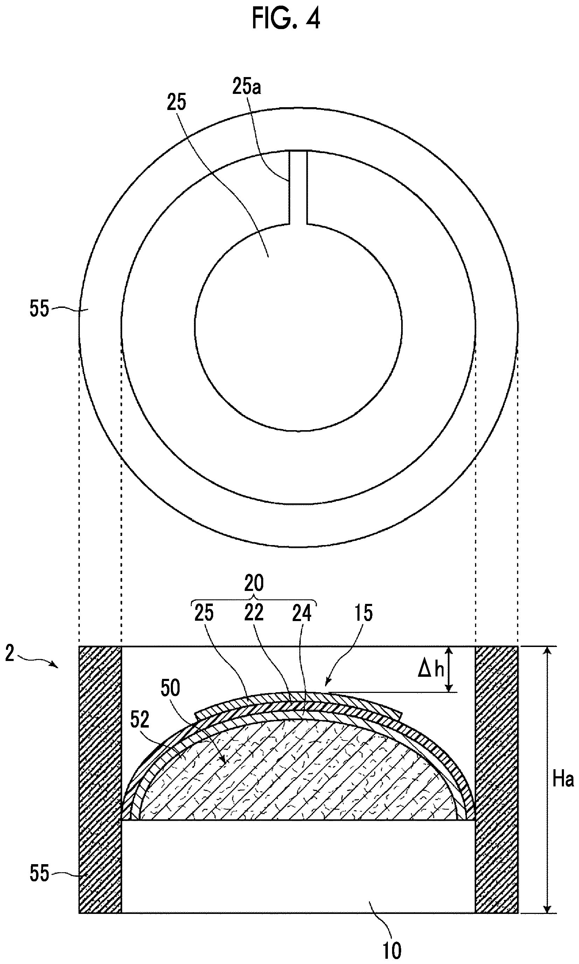

Fig. 4 is a schematic plan view showing a stethoscope 2 according to a second embodiment of the present invention and a schematic side view showing a part thereof as a cross-section. In Fig. 4, elements equivalent to the elements shown in Figs. 1 to 3 are designated by the same reference numerals, and detailed description thereof will not be repeated. The same applies to the following drawings.

The stethoscope 2 comprises a sound insulation member that blocks sounds from the outside (external sounds), such as environmental sounds and human voice, which is provided around a portion for detecting sounds generated from the object to be measured in the configuration of the stethoscope 1 of the first embodiment, that is, around the piezoelectric film 20 in the stethoscope 2. The sound insulation member preferably includes an elastic member for sound insulation having a portion protruding further outward than an apex of a convexly curved portion of the piezoelectric film 20. In the present embodiment, as the sound insulation member, an elastic member 55 for sound insulation having a portion protruding further outward than an apex of the convexly curved portion of the piezoelectric film 20 is provided. Other than that, the configuration is the same as that of the stethoscope 1. In Fig. 4, the detection circuit is not shown.

As shown in Fig. 4, the elastic member 55 is provided so as to protrude outward, that is, in a direction in which the distal end is away from the support base, to be Δh higher than the apex of a convexly curved portion of the piezoelectric film 20, that is, than the substantially center portion of the curved surface in the stethoscope 2.

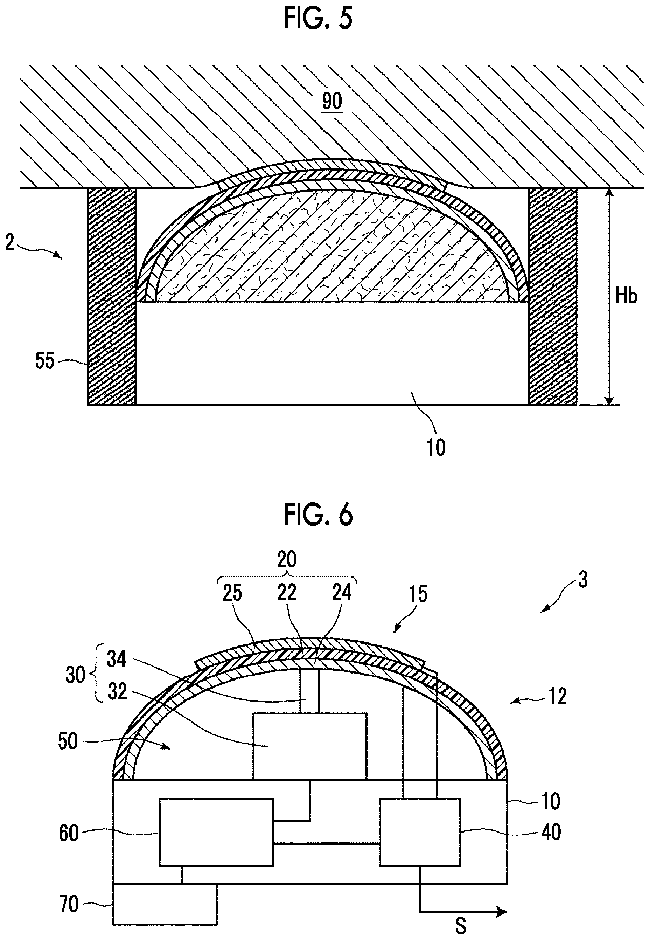

Fig. 5 shows a state in which the stethoscope 2 is pressed against the skin surface of a living body 90. As shown in Fig. 5, in a case of auscultating body sounds by using the stethoscope 2, the sensor region 15 provided with the second electrode 25 is pressed against the living body 90. In this case, the elastic member 55 comes into contact with the living body 90 before the second electrode 25 comes into contact with the living body 90, and the elastic member 55 is compressed until the second electrode 25 is pressed against the living body 90. In the stethoscope 2, in a state in which the sensor region 15 is not in contact with the living body 90, the thickness (height) of the elastic member 55 is denoted by Ha (see Fig. 4), and in a state in which the sensor region 15 is pressed against the living body 90, the elastic member 55 is compressed until the thickness thereof becomes Hb (< Ha). As shown in Fig. 5, in a case where the sensor region 15 is pressed against the living body 90, the sensor region 15 is surrounded by the living body 90 and the elastic member 55, so that the sounds from the outside such as environmental sounds can be blocked by the elastic member 55 and the influence of noise from the outside on the piezoelectric film 20 can be suppressed.

The elastic member 55 may be any member that is compressively deformable by at least Δh when the stethoscope 2 is pressed against the object to be measured. A part of the elastic member 55 may have an inelastic portion, and the other part thereof may have an elastic portion to be compressively deformable. As the elastic member 55, a material having a high absorption effect of environmental sounds, for example, a fiber-based material such as glass wool, and a foaming material such as urethane foam is preferably used. Here, the protruding height Δh of the elastic member 55 may be 0 mm or more, but is preferably 1 mm or more. It is preferable that the elastic member 55 is removable with respect to the detection unit 12. The sound insulation member is not limited to the elastic member, and a part thereof may form a Helmholtz resonator by using a slit or a perforated plate.

In the stethoscope having a structure in which the sensor region has a convex shape and a gap is generated around the portion coming into contact with the living body as in the stethoscope of the present disclosure, the elastic member 55 for sound insulation is provided as in the stethoscope 2 of the present embodiment, so that the noise due to environmental sounds can be suppressed.

Signals with a good SN ratio can be detected by pressing the piezoelectric film 20 against the object to be measured with an appropriate contact pressure by using the stethoscopes of the first and second embodiments. Since the displacement of the body surface caused by the body sounds such as heartbeat is extremely small, it is very important to press the piezoelectric film 20 against the living body with an appropriate pressure in order to acquire signals with a good SN ratio. Meanwhile, in applications such as home medical care and telemedicine, patients who do not have specialized skills may use the stethoscope. Therefore, it is desirable that the stethoscope is configured so that anyone can measure body sounds with an appropriate contact pressure and perform satisfactory detection.

### "Stethoscope of Third Embodiment"

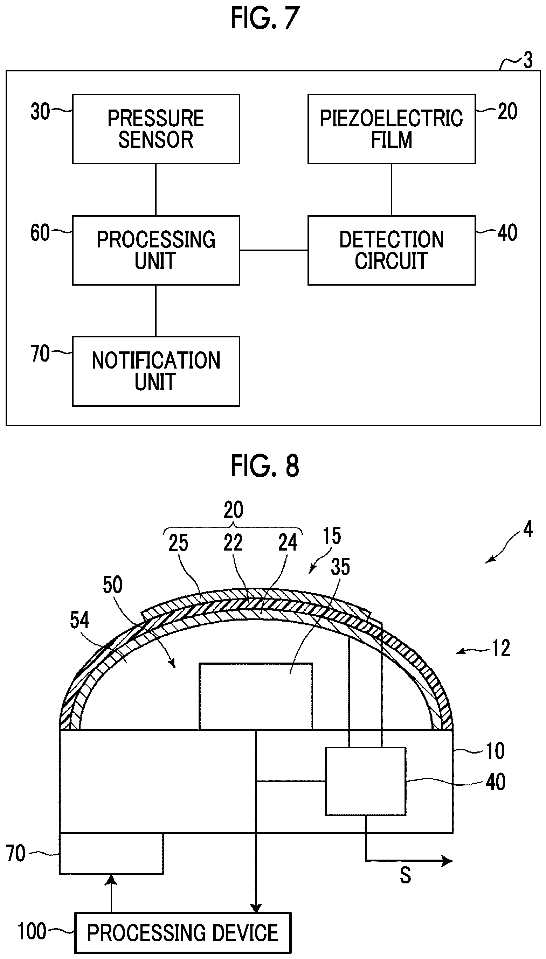

Fig. 6 is a schematic view of the stethoscope 3 according to a third embodiment of the present invention. Further, Fig. 7 is a block diagram showing the configuration of the stethoscope 3.

The stethoscope 3 of the third embodiment comprises a pressure sensor 30 for measuring the pressing force applied to the piezoelectric film 20 when the piezoelectric film 20 in the sensor region 15 is pressed against the living body, in the detection unit 12. The pressure sensor 30 comprises a main body 32 and a support portion 34. In a case where the external force is not applied to the piezoelectric film 20, the pressure sensor 30 exhibits a support function of supporting the piezoelectric film so that the piezoelectric film 20 is convexly curved outward by the support portion 34 disposed to be in contact with the first electrode 24 of the piezoelectric film 20. On the other hand, in a case where the piezoelectric film 20 is pressed against the living body or the like and a pressure exceeding the steady state in which the piezoelectric film 20 is supported by the support portion 34 is applied, the pressure sensor 30 detects the pressure as the pressing force applied to the piezoelectric film 20, that is, the contact pressure of the piezoelectric film 20 to the object to be measured. Further, in the stethoscope 3, the cushioning material is not provided in the space between the support base 10 and the piezoelectric film 20. In a case of comprising the pressure sensor 30 provided with the support portion 34 as described above, the piezoelectric film 20 is supported by the support portion 34, so that the cushioning material is not required.

As the pressure sensor 30, for example, a known sensor such as a spring scale, a strain gauge, or a diaphragm can be appropriately used.

The stethoscope 3 comprises a processing unit 60 that performs at least one of first processing of obtaining a contact pressure to the object to be measured having the maximum amplitude of the vibration signal detected with the piezoelectric film 20, or second processing of determining whether or not the contact pressure to the object to be measured is adequate.

Further, the stethoscope 3 of the present embodiment comprises a notification unit 70. The notification unit 70 has a function of notifying the user of at least one of that the contact pressure is optimal or that the contact pressure is within an adequate range. As the notification unit 70, for example, a lamp such as a light emitting diode (LED) or a buzzer can be used. The notification unit 70 may be provided anywhere as long as the notification unit 70 is provided at a location that does not hinder the operation of pressing the sensor region 15 against the object to be measured. In a case where the notification unit 70 is a visually recognizable unit such as a lamp, the sensor region 15 is preferably provided at a location easily visually recognizable in a state in which the sensor region 15 is pressed against the object to be measured.

The processing unit 60 performs processing of causing the notification unit 70 to make notification of at least one of the pressure obtained in the first processing or the determination result determined in the second processing.

The first processing is, for example, processing of obtaining a contact pressure at which the vibration signal having the maximum amplitude can be detected, from the vibration signals detected with the piezoelectric film 20 and data regarding the contact pressure detected by the pressure sensor 30. The user presses the sensor region 15 of the stethoscope 3 against the skin, gradually changes the contact pressure, and temporarily separates the stethoscope from the skin. The processing unit 60 obtains the contact pressure (optimal pressure) having the maximum amplitude from the relationship between the changing contact pressure and the vibration signal at each contact pressure. After that, in a case where the user presses the stethoscope 3 against the skin while gradually increasing the contact pressure again, when the contact pressure is the optimal pressure, the processing unit 60 causes the notification unit 70 to notify the user that the contact pressure is the optimal pressure by, for example, illuminating the lamp, changing the color of the lamp, or generating a sound. As a result, the user can measure body sounds such as heart sounds in a state in which the stethoscope is put to the skin at the optimal pressure.

The second processing is, for example, processing of determining whether or not the contact pressure is within the adequate contact pressure range provided in advance as data in the memory or the like, from the data regarding the contact pressure detected by the pressure sensor 30. In a case where the processing unit 60 determines that the contact pressure in a state in which the user presses the sensor region 15 of the stethoscope 3 against the skin is within the adequate contact pressure range, the processing unit 60 causes the notification unit 70 to notify the user that the contact pressure is adequate by illuminating the lamp, changing the color of the lamp, generating a sound, or the like. As a result, the user can measure body sounds such as heart sounds in a state in which the stethoscope is put to the skin at the appropriate contact pressure. The contact pressure is preferably about 1 kPa to 25 kPa.

The appropriate contact pressure varies depending on sounds as the measuring target, such as heart sounds, respiratory sounds, blood vessel sounds, or intestinal sounds. Therefore, it is preferable to provide data regarding the appropriate contact pressure range in advance for each sound as the measuring target so that the user specifies the measuring target when the measurement.

In the piezoelectric film 20, since sufficient displacement cannot be obtained in a state of being pressed with a strong contact pressure, the amplitude of the vibration signal may become small, and as a result, the sounds become low. In addition, since sufficient displacement does not occur in a state of being pressed with a weak contact pressure, the sounds also become low. Therefore, it is desirable to perform measurement with an appropriate pressure. With the stethoscope 3, the notification unit 70 can make the user easily confirm whether or not the stethoscope is in a state of being pressed with an adequate contact pressure, so that a person who does not have specialized skills can also perform measurement with a high SN ratio, which is more preferable.

The piezoelectric film 20 is suitable for measuring a dynamic pressure such as vibration, but is not suitable for measuring a static pressure such as a contact pressure in a case of pressing a stethoscope to listen to sounds. Therefore, it is preferable to comprise a separate static pressure sensor 30 as in the stethoscope 3 of the present embodiment.

Note that, since a pressure change of about 1 Hz or more can be detected in the piezoelectric film 20, it is also possible to have a configuration in which data regarding the contact pressure is detected even with only the piezoelectric film 20 without comprising the pressure sensor 30, as in the stethoscope 1. In the configuration of the stethoscope 1, a processing unit similar to the processing unit 60 of the stethoscope 3 may be provided, and the processing unit may be configured to acquire both the vibration signal and the data regarding the contact pressure from the piezoelectric film 20.

As described above, in the stethoscope 3, the correct sound can be sensed by sensing the pressure and notifying the user of an appropriate pressure range. In a case where there is a pressure sensing function, appropriate data can be acquired and the doctor can give appropriate instructions even in a case where the patient puts the stethoscope to his or her body in a remote diagnosis.

### "Stethoscope of Fourth Embodiment"

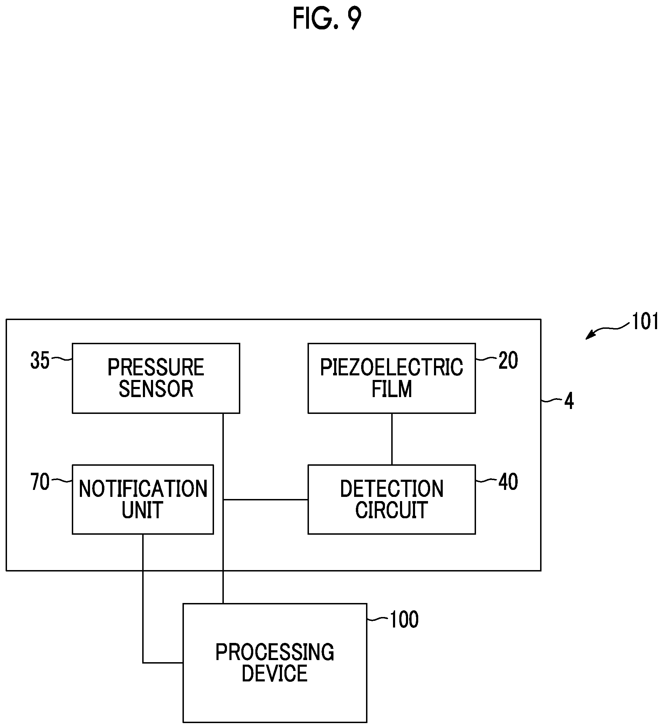

Fig. 8 is a schematic view of a stethoscope 4 according to a fourth embodiment of the present invention. Further, Fig. 9 is a block diagram showing a configuration of an electronic auscultation apparatus 101 including the stethoscope 4.

In the stethoscope 4, the space 50 between the piezoelectric film 20 and the support base 10 is filled with pressurized air 54 instead of the cushioning material. When the piezoelectric film 20 is pressed against the skin surface, a pressure sensor 35 detects that the air in the gap between the piezoelectric film 20 and the pressure sensor 35 is compressed and the pressure changes. The pressure change due to the compressed air corresponds to the change in the contact pressure of the piezoelectric film 20. As described above, in a case where the stethoscope is provided with the pressure sensor in the technique of the present disclosure, the pressure sensor may directly or indirectly measure the contact pressure of the piezoelectric film 20.

The stethoscope 4 of the present embodiment does not comprise a processing unit. However, the stethoscope 4 is connected to an external processing device 100 in a form capable of data communication. The connection form between the stethoscope 4 and the processing device 100 may be wireless or wired. The electronic auscultation apparatus 101 includes the stethoscope 4 and the processing device 100. Of course, the stethoscope 3 of the third embodiment can also be used with the processing device 100 connected thereto.

The vibration signal detected with the piezoelectric film 20 and the data regarding the contact pressure detected by the pressure sensor 35 are output from the stethoscope 4 to the processing device 100. The vibration signal detected with the piezoelectric film 20 may be output by being converted into a sound signal by the stethoscope 4, or may be output as a waveform on a monitor provided in the processing device 100.

The processing device 100 can be configured by a smartphone, a tablet computer, a personal computer, or the like in which a predetermined application is incorporated.

In the processing device 100, it is also possible to remove the noise from the data output from the stethoscope 4 by using an arithmetic circuit or a program and notify the user that the measurement can be performed with an optimal pressure value having a high SN ratio. In a case where the processing device 100 is provided with such a notification function, the stethoscope 4 may not be provided with the notification unit 70.

The processing device 100 has the same function as that of the processing unit 60 provided in the stethoscope 3 of the third embodiment. That is, the processing device 100 performs at least one of first processing of obtaining the contact pressure to the object to be measured having the maximum amplitude of the vibration signal detected with the piezoelectric film 20, or second processing of determining whether or not the contact pressure to the object to be measured is adequate. In addition, the processing device causes the notification unit 70 to make notification of whether or not the pressure obtained in the first processing and the contact pressure are adequate. Here, the first processing and the second processing are the same as the first processing and the second processing performed by the processing unit 60 provided in the stethoscope 3 of the third embodiment. Therefore, the same effect as with the case of the stethoscope 3 of the third embodiment can be obtained.

Further, the processing device 100 may record the vibration signals of the body sounds in daily measurement and data regarding the optimal pressure at which the maximum vibration signal can be obtained. With change in the optimal pressure, it is possible to diagnose changes in the body such as swelling of the body.

The processing device 100 may acquire the vibration signals and the data regarding the contact pressures continuously measured during the operation that the sensor region of the stethoscope is pressed against the skin and the stethoscope is separated from the skin while the user slowly changes the contact pressure, and extract and record data on the maximum amplitude of the vibration signal and data regarding the pressure when the vibration signal is detected. In this case, the processing device 100 can acquire a signal having a good SN ratio without performing the first and second processing.

### "Stethoscope of Fifth Embodiment"

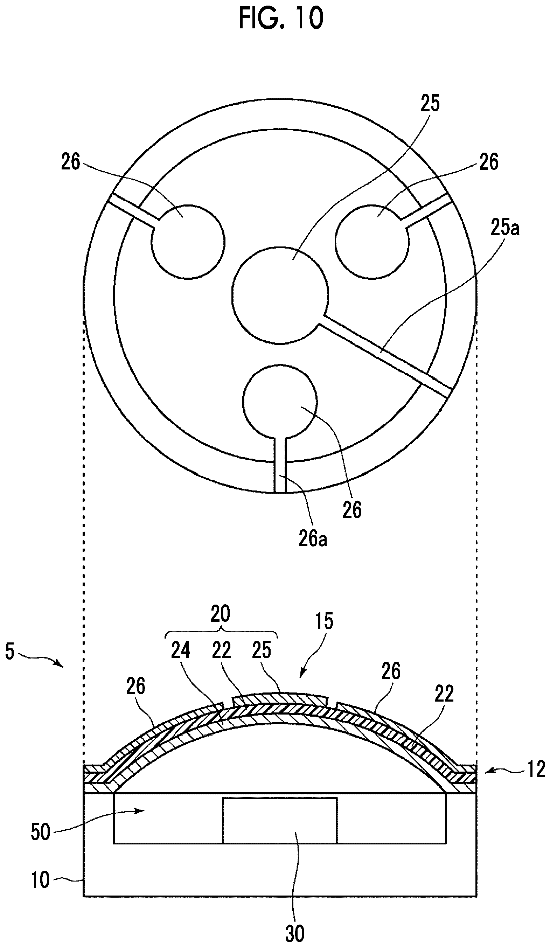

Fig. 10 is a schematic plan view and a schematic cross-sectional view showing a stethoscope 5 according to a fifth embodiment of the present disclosure. The fifth embodiment does not fall within the scope of the claimed subject-matter.

The stethoscope 5 comprises an electrocardiography electrode 26 in the detection unit 12. In the stethoscope 6 of the present embodiment, the electrocardiography electrode 26 is provided on the same surface as the second electrode 25 of the piezoelectric layer 22. In this case, it is preferable that the electrocardiography electrode 26 and the second electrode 25 each are formed of a patterned electrode layer. Here, the patterned electrode layer means that the electrode layer formed in the same process is formed by being subjected to patterning. A uniform electrode layer is formed on the piezoelectric layer 22 and is subjected to patterning, and thereby, the second electrode 25, the electrocardiography electrode 26, and wiring 25a and 26a thereof can be formed at the same time. In the present embodiment, three electrocardiography electrodes 26 arranged in a substantially equilateral triangle shape are provided, but the electrocardiography electrode 26 need only be two or more, and four or more electrocardiography electrodes may be provided. In Fig. 10, the detection circuit that detects the body sounds is not shown. The stethoscope 6 comprises a detection circuit for electrocardiography (not shown) in addition to the detection circuit for detecting body sounds. As shown in Fig. 10, the shape of the support base 10 may be a shape having a recess in a portion facing the piezoelectric film 20.

The heartbeat and the electrocardiogram can be measured at the same time by comprising the electrocardiography electrode 26. Since a plurality of data can be measured at the same time, the burden of the examination on the patient can be reduced.

The electrocardiography electrode 26 is not limited to an electrode formed of the patterned electrode layer that can be produced at the same time as the second electrode 25, and may be separately formed in a portion other than the sensor region 15 of the detection unit 12. Further, the electrocardiography electrode 26 may be provided on the surface side provided with the second electrode 25, through a protective layer provided on the second electrode 25.

### "Stethoscope of Sixth Embodiment"

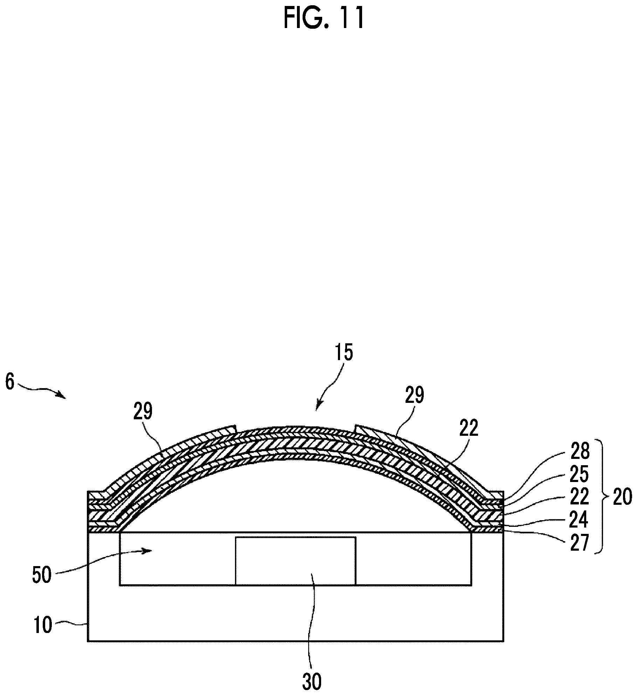

Fig. 11 is a schematic cross-sectional view of a stethoscope 6 according to a sixth embodiment of the present disclosure. The sixth embodiment does not fall within the scope of the claimed subject-matter.

As in the stethoscope 5 of the fifth embodiment, the stethoscope 6 comprises an electrocardiography electrode 29 in the detection unit 12. The configurations of the piezoelectric film 20 and the electrocardiography electrode 29 are different from the configuration of the stethoscope 6 of the sixth embodiment. The stethoscope 6 of the present embodiment comprises the piezoelectric film 20 having protective layers 27 and 28 on the surfaces of the first electrode 24 and the second electrode 25, respectively, as shown in Fig. 3B. The electrocardiography electrode 29 is provided on the surface of the protective layer 28 on the surface side of the piezoelectric film 20 provided with the second electrode 25. Since the electrocardiography electrode 29 is provided, the heartbeat and the electrocardiogram can be measured at the same time, as in the stethoscope 5 of the fifth embodiment. Since a plurality of data can be measured at the same time, the burden of the examination on the patient can be reduced. Further, since the piezoelectric film 20 provided with the protective layers 27 and 28 is provided, the effect of high durability is obtained.

In the stethoscopes 3 to 6 of the third to sixth embodiments, the sound insulation member such as the elastic member 55 for sound insulation for insulating the stethoscope from external sounds, which is provided in the stethoscope 2 of the second embodiment, can be provided and the noise from the outside can be suppressed by comprising the sound insulation member, which is preferable.

### "Confirmation Test Example"

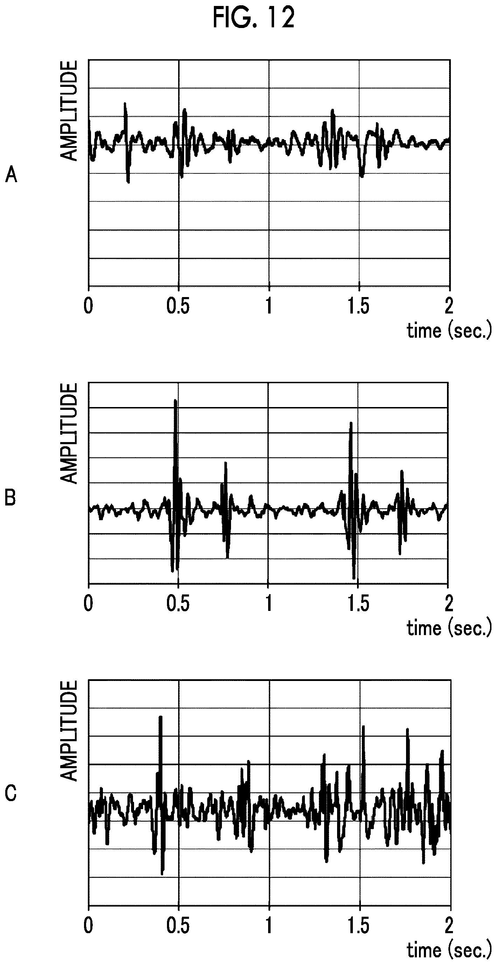

Fig. 12 shows data acquired by measuring the heartbeat by using the stethoscope 3 of the third embodiment shown in Fig. 6. A of Fig. 12 shows data in a case of being measured in a state in which the piezoelectric film is pressed against the living body at a pressure lower than the adequate contact pressure, B of Fig. 12 shows data in a case of being measured in a state in which the piezoelectric film is pressed against the living body at the adequate contact pressure, and C of Fig. 12 shows data in a case of being measured in a state in which the piezoelectric film is pressed against the living body at a pressure higher than the adequate contact pressure. In this example, the adequate pressure is about 3 kPa. Note that, the adequate pressure varies depending on the size of the stethoscope, the convex shape and the curvature of the curve of the piezoelectric film, and the like.

As shown in B of Fig. 12, it can be seen that a first sound and a second sound of the heartbeat can be clearly measured by acquiring the signal with the contact pressure at which the maximum amplitude can be obtained. On the other hand, as shown in A of Fig. 12, in a case of acquiring the signal at a pressure lower than the adequate contact pressure, waveform data in which the amplitude of the signal is relatively small and a lot of noise components exist as compared with the case of the adequate pressure, is shown. It is considered that this is because the measurement surface is not sufficiently in contact with the body surface. Further, as shown in C of Fig. 12, in a case of acquiring the signal at a pressure higher than the adequate contact pressure, waveform data in which a lot of noise components exist as with the case of the low pressure, is shown.

As shown in Fig. 12, it is clarified that body sounds can be acquired with a very high SN ratio by pressing the stethoscope according to the present disclosure against the skin at an adequate contact pressure.

### Explanation of References

1, 2, 3, 4, 5, 6: stethoscope
10: support base
12: detection unit
15: sensor region
20: piezoelectric film
22: piezoelectric layer
22a: matrix
22b: piezoelectric particle
24: first electrode
25: second electrode
25a: wiring
26, 29: electrocardiography electrode
26a: wiring
27, 28: protective layer
30, 35, 130: pressure sensor
32: main body
34: support portion
40: detection circuit
50: space
52: cushioning material
54: pressurized air
55: elastic member for sound insulation
60: processing unit
70: notification unit
90: living body
100: processing device
101: electronic auscultation apparatus

## Claims

1. A stethoscope comprising:
a support base (10); and
a detection unit (12) that is supported by the support base (10) and detects a sound generated from an object to be measured,
wherein the detection unit (12) has a piezoelectric film (20) that is disposed to face the support base (10) in at least a portion for detecting the sound generated from the object to be measured and that is convexly curved to a side opposite to the support base (10),
the piezoelectric film (20) includes a piezoelectric layer (22) having two main surfaces facing each other, a first electrode (24) provided on a main surface on a support base side of the two main surfaces, and a second electrode (25) provided on a main surface on a side opposite to the support base (10),
wherein
a strain generated in the piezoelectric film (20) due to the sound generated from the object to be measured is detected as a vibration signal,
**characterised in that** the second electrode (25) is provided only in a central part of a convexly curved portion of the piezoelectric film (20).

2. The stethoscope according to claim 1, further comprising:
a sound insulation member that blocks an external sound, which is provided around the portion for detecting the sound generated from the object to be measured.

3. The stethoscope according to claim 2,
wherein the sound insulation member is formed of an elastic member (55) that has a portion protruding further outward than an apex of a convexly curved portion of the piezoelectric film (20).

4. The stethoscope according to any one of claims 1 to 3, further comprising:
a cushioning material (52) between the support base (10) and the piezoelectric film (20).

5. The stethoscope according to any one of claims 1 to 3, further comprising:
a pressurized gas (54) in a space (50) between the support base (10) and the piezoelectric film (20).

6. The stethoscope according to any one of claims 1 to 5,
wherein the piezoelectric layer (22) consists of a polymer composite piezoelectric body obtained by dispersing piezoelectric particles (22b) in a matrix (22a) consisting of a polymer material.

7. The stethoscope according to any one of claims 1 to 6, further comprising:
an electrocardiography electrode (26, 29).

8. The stethoscope according to claim 7,
wherein the electrocardiography electrode (26, 29) is provided on a surface side of the piezoelectric film (20) provided with the second electrode (25).

9. The stethoscope according to claim 8,
wherein the electrocardiography electrode (26, 29) and the second electrode (25) each are formed of a patterned electrode layer.

10. The stethoscope according to any one of claims 1 to 9, further comprising:
a pressure sensor (30, 35, 130) that detects a contact pressure of the piezoelectric film (20) to the obj ect to be measured, between the support base (10) and the piezoelectric film (20).

11. The stethoscope according to any one of claims 1 to 1 0, further comprising:
a processing unit (60) that performs at least one of first processing of obtaining a contact pressure to the object to be measured having a maximum amplitude of the vibration signal detected with the piezoelectric film (20) or second processing of determining whether or not a contact pressure to the object to be measured is adequate.

12. The stethoscope according to claim 11,
wherein the processing unit (60) performs processing of causing a notification unit (70) to make notification of at least one of the pressure obtained in the first processing or a determination result determined in the second processing.

13. An electronic auscultation apparatus (101) comprising:
the stethoscope according to any one of claims 1 to 12; and
a processing device (100) that receives a vibration signal and data regarding a contact pressure detected by the stethoscope,
wherein the processing device (100) performs at least one of first processing of obtaining a contact pressure to the object to be measured having a maximum amplitude of the vibration signal detected with the piezoelectric film (20) or second processing of determining whether or not a contact pressure to the object to be measured is adequate, from the vibration signal and the data regarding the contact pressure.

## Patentansprüche

1. Stethoskop, umfassend:
eine Trägerbasis (10); und
eine Erfassungseinheit (12), die von der Trägerbasis (10) getragen wird und einen von einem zu messenden Objekt erzeugten Schall erfasst,
wobei die Erfassungseinheit (12) einen piezoelektrischen Film (20) aufweist, der so angeordnet ist, dass er der Trägerbasis (10) in mindestens einem Teil zum Erfassen des von dem zu messenden Objekt erzeugten Schalls zugewandt ist, und der zu einer der Trägerbasis (10) gegenüberliegenden Seite konvex gekrümmt ist,
der piezoelektrische Film (20) eine piezoelektrische Schicht (22) mit zwei einander zugewandten Hauptoberflächen, eine erste Elektrode (24), die auf einer Hauptoberfläche auf einer Trägerbasisseite der beiden Hauptoberflächen vorgesehen ist, und eine zweite Elektrode (25), die auf einer Hauptoberfläche auf einer der Trägerbasis (10) gegenüberliegenden Seite vorgesehen ist, beinhaltet, wobei
eine Spannung, die in dem piezoelektrischen Film (20) aufgrund des von dem zu messenden Objekt erzeugten Schalls erzeugt wird, als ein Vibrationssignal erfasst wird, **dadurch gekennzeichnet, dass** die zweite Elektrode (25) nur in einem zentralen Teil eines konvex gekrümmten Teils des piezoelektrischen Films (20) vorgesehen ist.

2. Stethoskop nach Anspruch 1, weiter umfassend:
ein schalldämmendes Element, das einen Außenschall blockiert und das um den Teil zur Erfassung des von dem zu messenden Objekt erzeugten Schalls angeordnet ist.

3. Stethoskop nach Anspruch 2,
wobei das schalldämmende Element aus einem elastischen Element (55) gebildet ist, das einen Teil aufweist, der weiter nach außen als ein Scheitelpunkt eines konvex gekrümmten Teils des piezoelektrischen Films (20) vorsteht.

4. Stethoskop nach einem der Ansprüche 1 bis 3, weiter umfassend:
ein Polstermaterial (52) zwischen der Trägerbasis (10) und dem piezoelektrischen Film (20).

5. Stethoskop nach einem der Ansprüche 1 bis 3, weiter umfassend:
ein Druckgas (54) in einem Raum (50) zwischen der Trägerbasis (10) und dem piezoelektrischen Film (20).

6. Stethoskop nach einem der Ansprüche 1 bis 5,
wobei die piezoelektrische Schicht (22) aus einem piezoelektrischen Polymerverbundkörper besteht, der durch Dispergieren von piezoelektrischen Teilchen (22b) in einer aus einem Polymermaterial bestehenden Matrix (22a) erhalten wird.

7. Stethoskop nach einem der Ansprüche 1 bis 6, weiter umfassend:
eine Elektrokardiographie-Elektrode (26, 29).

8. Stethoskop nach Anspruch 7,
wobei die Elektrokardiographie-Elektrode (26, 29) auf einer Oberflächenseite des mit der zweiten Elektrode (25) versehenen piezoelektrischen Films (20) vorgesehen ist.

9. Stethoskop nach Anspruch 8,
wobei die Elektrokardiographie-Elektrode (26, 29) und die zweite Elektrode (25) jeweils aus einer strukturierten Elektrodenschicht gebildet sind.

10. Stethoskop nach einem der Ansprüche 1 bis 9, weiter umfassend:
einen Drucksensor (30, 35, 130), der einen Anpressdruck des piezoelektrischen Films (20) auf das zu messende Objekt zwischen der Trägerbasis (10) und dem piezoelektrischen Film (20) erfasst.

11. Stethoskop nach einem der Ansprüche 1 bis 10, weiter umfassend:
eine Verarbeitungseinheit (60), die mindestens eine von einer ersten Verarbeitung zum Erhalten eines Anpressdrucks auf das zu messende Objekt mit einer maximalen Amplitude des mit dem piezoelektrischen Film (20) erfassten Vibrationssignals oder einer zweiten Verarbeitung zum Bestimmen, ob ein Anpressdruck auf das zu messende Objekt ausreichend ist, durchführt.

12. Stethoskop nach Anspruch 11,
wobei die Verarbeitungseinheit (60) eine Verarbeitung durchführt, die eine Benachrichtigungseinheit (70) veranlasst, eine Benachrichtigung über mindestens einen von den in der ersten Verarbeitung erhaltenen Druck und/oder ein in der zweiten Verarbeitung ermitteltes Bestimmungsergebnis vorzunehmen.

13. Elektronische Abhöreinrichtung (101), umfassend:
das Stethoskop nach einem der Ansprüche 1 bis 12; und
eine Verarbeitungsvorrichtung (100), die ein Vibrationssignal und Daten über einen vom Stethoskop erfassten Anpressdruck empfängt,
wobei die Verarbeitungsvorrichtung (100) mindestens eine von einer ersten Verarbeitung zum Erhalten eines Anpressdrucks auf das zu messende Objekt mit einer maximalen Amplitude des mit dem piezoelektrischen Film (20) erfassten Vibrationssignals oder eine zweite Verarbeitung zum Bestimmen, ob ein Anpressdruck auf das zu messende Objekt ausreichend ist, anhand des Vibrationssignals und der Daten über den Anpressdruck durchführt.

## Revendications

1. Stéthoscope comprenant :
une base de support (10) ; et
une unité de détection (12) qui est soutenue par la base de support (10) et détecte un son généré par un objet à mesurer,
dans lequel l'unité de détection (12) présente un film piézoélectrique (20) qui est disposé de manière à faire face à la base de support (10) dans au moins une portion pour détecter le son généré par l'objet à mesurer et qui est incurvé de manière convexe vers un côté opposé à la base de support (10),
le film piézoélectrique (20) inclut une couche piézoélectrique (22) présentant deux surfaces principales se faisant face, une première électrode (24) fournie sur une surface principale sur un côté base de support des deux surfaces principales, et une seconde électrode (25) fournie sur une surface principale sur un côté opposé à la base de support (10), dans lequel
une contrainte générée dans le film piézoélectrique (20) en raison du son généré par l'objet à mesurer est détectée comme signal de vibration, **caractérisé en ce que** la seconde électrode (25) est uniquement fournie dans une partie centrale d'une portion incurvée de manière convexe du film piézoélectrique (20).

2. Stéthoscope selon la revendication 1, comprenant en outre :
un élément d'isolation acoustique qui bloque un son externe, qui est fourni autour de la portion destinée à détecter le son généré par l'objet à mesurer.

3. Stéthoscope selon la revendication 2,
dans lequel l'élément d'isolation acoustique est formé d'un élément élastique (55) qui présente une portion faisant saillie davantage vers l'extérieur qu'un sommet d'une portion incurvée de manière convexe du film piézoélectrique (20).

4. Stéthoscope selon l'une quelconque des revendications 1 à 3, comprenant en outre :
un matériau de rembourrage (52) entre la base de support (10) et le film piézoélectrique (20).

5. Stéthoscope selon l'une quelconque des revendications 1 à 3, comprenant en outre :
un gaz sous pression (54) dans un espace (50) compris entre la base de support (10) et le film piézoélectrique (20).

6. Stéthoscope selon l'une quelconque des revendications 1 à 5,
dans lequel la couche piézoélectrique (22) consiste en un corps piézoélectrique composite polymère obtenu en dispersant des particules piézoélectriques (22b) dans une matrice (22a) consistant en un matériau polymère.

7. Stéthoscope selon l'une quelconque des revendications 1 à 6, comprenant en outre :
une électrode d'électrocardiographie (26, 29).

8. Stéthoscope selon la revendication 7,
dans lequel l'électrode d'électrocardiographie (26, 29) est fournie sur un côté surface du film piézoélectrique (20) muni de la seconde électrode (25).

9. Stéthoscope selon la revendication 8,
dans lequel l'électrode d'électrocardiographie (26, 29) et la seconde électrode (25) sont chacune formées d'une couche d'électrode à motifs.

10. Stéthoscope selon l'une quelconque des revendications 1 à 9, comprenant en outre :
un capteur de pression (30, 35, 130) qui détecte une pression de contact du film piézoélectrique (20) sur l'objet à mesurer, entre la base de support (10) et le film piézoélectrique (20).

11. Stéthoscope selon l'une quelconque des revendications 1 à 10, comprenant en outre :
une unité de traitement (60) qui effectue au moins l'un parmi un premier traitement pour obtenir une pression de contact sur l'objet à mesurer présentant une amplitude maximale du signal de vibration détecté avec le film piézoélectrique (20) ou un second traitement pour déterminer si une pression de contact sur l'objet à mesurer est suffisante ou non.

12. Stéthoscope selon la revendication 11,
dans lequel l'unité de traitement (60) effectue un traitement pour amener une unité de notification (70) à notifier au moins l'un parmi la pression obtenue lors du premier traitement ou un résultat de détermination déterminé lors du second traitement.

13. Appareil d'auscultation électronique (101) comprenant :
le stéthoscope selon l'une quelconque des revendications 1 à 12 ; et
un dispositif de traitement (100) qui reçoit un signal de vibration et des données concernant une pression de contact détectée par le stéthoscope,
dans lequel le dispositif de traitement (100) effectue au moins l'un parmi un premier traitement pour obtenir une pression de contact sur l'objet à mesurer présentant une amplitude maximale du signal de vibration détecté avec le film piézoélectrique (20) ou un second traitement pour déterminer si une pression de contact sur l'objet à mesurer est suffisante ou non, à partir du signal de vibration et des données concernant la pression de contact.
